# FASCICULE DE BREVET EUROPEEN

(11) **EP 1 110 934 B1**
(45) Date de publication et mention de la délivrance du brevet: **04.06.2003**
(21) Numéro de dépôt: 00403479.9
(22) Date de dépôt: 11.12.2000
(51) Int. Cl.: C07C 11/09, C07C 11/06

(54) **Production d'isobutène de haute pureté et de propylène à partir de coupes d'hydrocarbures à quatre atomes de carbone**
Herstellung von sehr reinem Isobuten und von Propen aus Kohlenwasserstoffschnitten mit vier Kohlenstoffatomen
Preparation of very pure isobutene and of propylene from hydrocarbon cuts with four carbon atoms

(30) Priorité: 24.12.1999 FR 9916507
(43) Date de publication de la demande: 27.06.2001
(73) Titulaire: Institut Francais du Petrole, 92852 Rueil-Malmaison Cedex (FR)
(72) Inventeur: Commereuc, Dominique, 92190 Meudon (FR); Didillon, Blaise, 69340 Francheville (FR); Olivier, Hélène, 92500 Rueil Malmaison (FR); Saussine, Lucien, 78290 Croissy sur Seine (FR)

(56) Documents cités:
- FR-A- 2 755 130

## Description

L'invention concerne un procédé de production d'isobutène de haute pureté et de propylène à partir d'une coupe C₄.

Le vapocraquage de charges constituées par des coupes paraffiniques légères fournit l'éthylène et le propylène nécessaires à la pétrochimie. Il fournit également un certain nombre d'autres produits plus lourds, et en particulier une coupe d'hydrocarbures en C₄ qui contient principalement du butadiène-1,3, de l'isobutène, des n-butènes et des butanes, accompagnés de traces d'hydrocarbures acétyléniques.

Le craquage catalytique de charges d'hydrocarbures lourds fournit, à côté des fractions essence et gazole qui sont les produits principaux, des produits plus légers, parmi lesquels une coupe d'hydrocarbures en C₄ qui contient principalement de l'isobutane, de l'isobutène, des n-butènes et des butanes, accompagnés de faibles quantités de butadiène-1,3 et d'hydrocarbures acétyléniques.

Jusqu'à récemment, seuls le butadiène-1,3 et l'isobutène trouvaient un usage dans l'industrie des polymères, en particulier dans l'industrie des pneumatiques. L'augmentation de la longévité des pneumatiques et une relative stagnation de la demande font qu'il existe maintenant des surplus de butadiène qui ne sont pas, ou mal, utilisés. Au contraire, il y a un regain d'intérêt pour l'isobutène qui peut être utilisé par exemple comme monomère dans la synthèse de polyisobutène.

La présente invention propose un procédé de traitement d'une coupe d'hydrocarbures en C₄ contenant principalement de l'isobutène, des n-butènes, des butanes, et du butadiène-1,3 en quantité variable, qui inclut la séparation de l'isobutène au moyen d'une distillation catalytique dans laquelle le butène-1 qu'il est impossible de séparer de l'isobutène est isomérisé en butènes-2, et qui permet de transformer le butadiène-1,3 et les n-butènes en propylène utilisable par exemple pour la polymérisation.

Les proportions relatives d'éthylène et de propylène produits dans une opération de vapocraquage peuvent être modulées dans une certaine mesure en changeant la nature de la charge et en modifiant les conditions opératoires (la sévérité) du craquage. Cependant, le mode de fonctionnement orienté vers une proportion plus grande de propylène entraîne inévitablement une baisse du rendement en éthylène et une production plus importante de coupe C₄ et de fraction essence.

Un autre objet de la présente invention est d'augmenter la production de propylène tout en maintenant un haut rendement en éthylène grâce au traitement de la coupe d'hydrocarbures en C₄ et donc sans que l'on soit obligé de baisser la sévérité du vapocraqueur.

Le procédé objet de l'invention est plus précisément un procédé de de conversion d'une coupe C₄ oléfinique en isobutène de haute pureté et en propylène, ladite coupe contenant notamment des dioléfines, du butène-1, des butènes-2, de l'isobutène et des impuretés acétyléniques, ledit procédé comportant les étapes suivantes, se déroulant successivement :
1) l'hydrogénation sélective des dioléfines et des impuretés acétyléniques avec isomérisation du butène-1 en butènes-2 en présence d'un catalyseur, de façon à obtenir un effluent contenant des n-butènes dont le ratio correspond à l'équilibre thermodynamique et de l'isobutène, et ne contenant pratiquement pas de dioléfines ni de composés acétyléniques ;
2) la séparation, par distillation, d'une coupe de tête contenant l'isobutène et d'une coupe de pied contenant essentiellement les butènes-2 et le butane ; et
3) la métathèse de la coupe butènes-2 issue de l'étape précédente avec de l'éthylène, en présence d'un catalyseur, de façon à obtenir un effluent contenant du propylène, la métathèse étant suivie d'une séparation du propylène,
ledit procédé étant caractérisé en ce que l'étape 2 est mise en oeuvre dans une colonne intégrant l'hydro-isomérisation du butène-1 restant en butènes-2.

On décrira ci-après plus en détail les conditions particulières des différentes étapes du procédé selon l'invention réalisé à partir d'une coupe d'hydrocarbures en C₄ qui contient principalement de l'isobutène, des n-butènes, des butanes, ainsi que du butadiène en quantité variable, ladite coupe C₄ étant soumise à ces étapes pour produire de l'isobutène et du propylène.

L'objet principal de la première étape est de transformer le butadiène et le butène-1 en butènes-2. En effet, les butènes-2 sont la source du propylène produit dans la dernière étape de métathèse en présence d'éthylène. Il est donc souhaitable d'accroître le plus possible le rendement en butènes-2, c'est-à-dire de se rapprocher autant que possible de la proportion autorisée par la thermodynamique. Le deuxième objet de cette étape est d'éliminer les traces d'hydrocarbures acétyléniques toujours présentes dans ces coupes et qui sont des poisons ou des polluants pour les étapes ultérieures.

Dans cette première étape, on réalise donc simultanément les réactions suivantes, en présence d'hydrogène:
- l'hydrogénation sélective du butadiène en un mélange de n-butènes à l'équilibre thermodynamique,
- l'isomérisation du butène-1 en butènes-2, de manière à obtenir une répartition proche de l'équilibre thermodynamique des n-butènes, et
- l'hydrogénation sélective des traces d'hydrocarbures acétyléniques en butènes.

Ces réactions peuvent être réalisées au moyen de divers catalyseurs spécifiques comprenant un ou plusieurs métaux, par exemple du groupe 10 de la classification périodique (Ni, Pd ou Pt), déposés sur un support. On met en oeuvre de préférence un catalyseur qui comprend au moins un composé de palladium fixé sur un support minéral réfractaire, par exemple sur une alumine. La teneur en palladium sur le support peut être de 0,01 à 5 % en poids, de préférence de 0,05 à 1 % en poids. Divers modes de pré-traitement connus de l'homme du métier peuvent éventuellement être appliqués à ces catalyseurs pour en améliorer la sélectivité dans l'hydrogénation du butadiène en butènes aux dépens de l'hydrogénation totale en butane, qu'il faut éviter, et pour favoriser l'hydroisomérisation des n-butènes (du butène-1 en butènes-2). De préférence, le catalyseur contient de 0,05 à 10 % en poids de soufre. De façon avantageuse, on utilise un catalyseur constitué par du palladium déposé sur de l'alumine, et du soufre.

Le catalyseur peut avantageusement être mis en oeuvre selon le procédé décrit dans le brevet FR-B-2 708 596, c'est-à-dire que le catalyseur a été traité, avant son chargement dans le réacteur d'hydrogénation, par au moins un composé soufré dilué dans un solvant, puis le catalyseur obtenu, contenant de 0,05 à 10 % en poids de soufre, est chargé dans le réacteur et activé sous atmosphère neutre ou réductrice à une température de 20 à 300 °C, une pression de 0,1 à 5 MPa et une VVH de 50 à 600 h⁻¹, et la charge est mise au contact dudit catalyseur activé.

La mise en oeuvre du catalyseur, de préférence au palladium, n'est pas critique, mais on préfère généralement utiliser au moins un réacteur à flux descendant à travers un lit fixe de catalyseur. Lorsque la proportion de butadiène dans la coupe est importante, ce qui est le cas par exemple d'une coupe de vapocraquage lorsqu'on ne souhaite pas en extraire le butadiène pour des usages spécifiques, il peut être avantageux d'effectuer la transformation dans deux réacteurs en série afin de mieux contrôler la sélectivité de l'hydrogénation. Le second réacteur peut être à flux ascendant et avoir un rôle de finition.

La quantité d'hydrogène nécessaire à l'ensemble des réactions réalisées dans cette étape est ajustée en fonction de la composition de la coupe pour avoir avantageusement seulement un léger excès d'hydrogène par rapport à la stoechiométrie.

Les conditions opératoires sont choisies de telle façon que les réactifs et les produits soient en phase liquide. Il peut cependant être avantageux de choisir un mode de fonctionnement tel que les produits soient partiellement vaporisés à la sortie du réacteur, ce qui facilite le contrôle thermique de la réaction. La température peut varier de 20 à 200 °C, de préférence de 50 à 150 °C ou mieux de 60 à 100 °C. La pression peut être ajustée à une valeur de 0,1 à 5 MPa, de préférence de 0,5 à 4 MPa et avantageusement de 0,5 à 3 MPa, de telle façon que les réactifs, au moins en partie, soient en phase liquide. La vitesse spatiale peut être de 0,5 à 20 h⁻¹ et de préférence de 1 à 10 h⁻¹, avec un rapport molaire H₂ / dioléfines de 0,5 à 5 et de préférence de 1 à 3.

Le ou les réacteurs utilisés pour mettre en oeuvre l'étape 1 d'hydrogénation sélective et d'isomérisation peuvent avantageusement être suivis d'une colonne de stabilisation, qui élimine les traces d'hydrocarbures gazeux éventuellement présents dans l'hydrogène de charge.

L'objet de la deuxième étape est de séparer, dans une colonne intégrant l'hydro-isomérisation des n-butènes (c'est-à-dire du butène-1 restant après l'étape 1 en butènes-2), la coupe C₄ issue de l'étape 1, pour obtenir d'une part et tête une fraction contenant essentiellement de l'isobutène exempt de butène-1, d'autre part en pied une fraction contenant une faible quantité de butène-1, les butènes-2 et le n-butane. L'isobutène ainsi concentré peut être destiné à des usages variés. La fraction butènes-2 est dirigée vers l'étape de métathèse. En général, la coupe de pied de cette étape contient au plus 1 % en poids d'isobutène et au plus 1 % en poids de butène-1.

La colonne intégrant l'hydro-isomérisation des n-butènes comporte, intérieurement ou extérieurement, une ou plusieurs charges d'un catalyseur du même type que celui mis en oeuvre dans l'étape 1. La colonne intégrant l'hydro-isomérisation des n-butènes employée dans le procédé selon l'invention peut être de n'importe quel type. Dans une disposition préférée, au moins une zone contenant le catalyseur est aménagée. La disposition mécanique du catalyseur dans la ou les zones catalytiques doit être telle qu'elle gêne le moins possible les flux de vapeur et de liquide entre les deux zones de séparation qui l'encadrent. Le catalyseur peut par exemple être disposé en couche mince sur des plateaux perforés ou sur des grilles, ou dans des sachets suspendus ou posés sur des supports assurant sa tenue mécanique, ou de toute autre façon connue de l'homme de l'art. D'autre part, le catalyseur peut être disposé dans la colonne de manière à n'être traversé que par un flux ascendant de phase liquide. Il peut également être disposé sous forme de garnissage catalytique, suivant les différents modes de mise en oeuvre connus. Les zones de séparation qui encadrent les zones catalytiques peuvent comporter des plateaux ou du garnissage. Une des mises en oeuvre de la colonne peut par exemple correspondre au brevet FR-B-2 755 130, au nom de la demanderesse.

La fraction butènes-2 issue de l'étape 2 peut être envoyée directement dans la troisième étape du procédé. Dans cette dernière étape, les butènes-2 sont mis en réaction avec de l'éthylène, pour donner du propylène par métathèse. En raison de la faible quantité de butène-1 et d'isobutène dans la charge, la formation de sous-produits est très limitée.

La réaction de métathèse de l'éthylène avec les butènes-2 dans l'étape 3 peut être catalysée par des oxydes métalliques variés déposés sur des supports. On utilise de préférence un catalyseur comportant au moins un oxyde de rhénium déposé sur un support composé par un oxyde réfractaire contenant au moins de l'alumine, qui présente un caractère acide, comme par exemple l'alumine elle-même, les silices-alumines ou les zéolithes.

On peut citer à titre d'exemples préférés les catalyseurs comportant de l'heptoxyde de rhénium déposé sur une alumine gamma analogue à celle utilisée dans les catalyseurs de reforming, comme décrits dans le brevet US-A-4 795 734. La teneur en rhénium (exprimée en rhénium métallique) peut être de 0,01 et 20%, de préférence de 1 à 15% en poids. Les catalyseurs sont soumis par exemple à une activation thermique finale à une température de 400 à 1000 °C pendant une durée de 10 minutes à 5 heures sous une atmosphère non-réductrice.

Les catalyseurs comportant de l'heptoxyde de rhénium déposé sur une alumine peuvent aussi être modifiés par l'adjonction d'un oxyde d'un autre métal. De tels catalyseurs modifiés comprennent par exemple du rhénium à l'état d'oxyde, de 0,01 à 20 % en poids exprimé en rhénium métallique, déposé sur un support contenant au moins 75 % en poids d'alumine et de 0,01 à 30 % en poids d'au moins un oxyde d'un métal choisi dans le groupe formé par le niobium et le tantale, tel que décrit dans le brevet FR-B-2 709 125.

La réaction de métathèse est effectuée de préférence en phase liquide, en l'absence d'oxygène, de composés oxygénés et d'humidité, et à une température de 0 à 200 °C, de préférence de 20 à 150 °C, sous une pression au moins égale à la tension de vapeur du mélange réactionnel à la température de réaction.

Le catalyseur peut être mis en oeuvre en lit fixe. Cependant, comme il doit être fréquemment régénéré, il est en général nécessaire de disposer d'au moins deux réacteurs en parallèle, l'un étant en fonctionnement pendant que l'autre est en régénération. On utilise de préférence un système de lit catalytique mobile comme il est décrit dans le brevet français FR-B-2 608 595. Le catalyseur est soutiré à intervalles de temps réguliers en bas du réacteur et transféré vers un système de régénération en continu, d'où il est renvoyé en haut du réacteur.

Compte tenu des limitations imposées par la thermodynamique, l'éthylène non converti est fractionné dans une première colonne de distillation et recyclé au réacteur de métathèse. Une deuxième colonne de distillation sépare le propylène et les hydrocarbures en C₄ non convertis qui peuvent être recyclés au réacteur de métathèse. Le schéma de fractionnement est donc plus simple que si une grande quantité de butène-1 avait été présente dans la charge, car il se serait alors formé davantage de pentènes et d'hexènes, qu'il aurait fallu éliminer avant de recycler les butènes.

Lorsque le procédé est appliqué à une coupe C₄ de vapocraquage, il peut être avantageux d'intégrer l'unité de métathèse avec le craqueur, de manière à bénéficier du train de fractionnement de celui-ci. On utilise alors dans l'étape de métathèse de l'éthylène provenant de l'opération de vapocraquage.

La succession des traitements retenue dans le procédé selon l'invention présente de nombreux avantages. Les composés les plus réactifs de la coupe, en particulier le butadiène-1,3, présent en quantité variable, ainsi que les traces d'hydrocarbures acétyléniques sont transformés dès la première étape, et ne seront donc pas la cause de réactions parasites dans les étapes suivantes. Par ailleurs l'hydrogénation sélective des dioléfines (butadiène-1,3 et -1,2) en butènes et l'hydro-isomérisation du butène-1 couplée à la séparation dans une colonne intégrant l'hydro-isomérisation des n-butènes permet d'augmenter considérablement la concentration en butènes-2 dans la coupe tout en réduisant à des valeurs faibles la concentration en butène-1, ce qui favorise d'autant un rendement élevé en propylène dans l'étape de métathèse.

En effet, le butène-1 réagit en métathèse avec les butènes-2 pour donner du propylène et des pentènes, et il réagit sur lui-même pour donner des hexènes. Pentènes et hexènes sont des sous-produits de faible valeur dont il faut se débarrasser de manière coûteuse. Le procédé permet donc une augmentation appréciable du rendement en propylène, et facilite le recyclage des butènes-2 au réacteur de métathèse, puisqu'il y a peu de pentènes et d'hexènes à éliminer.

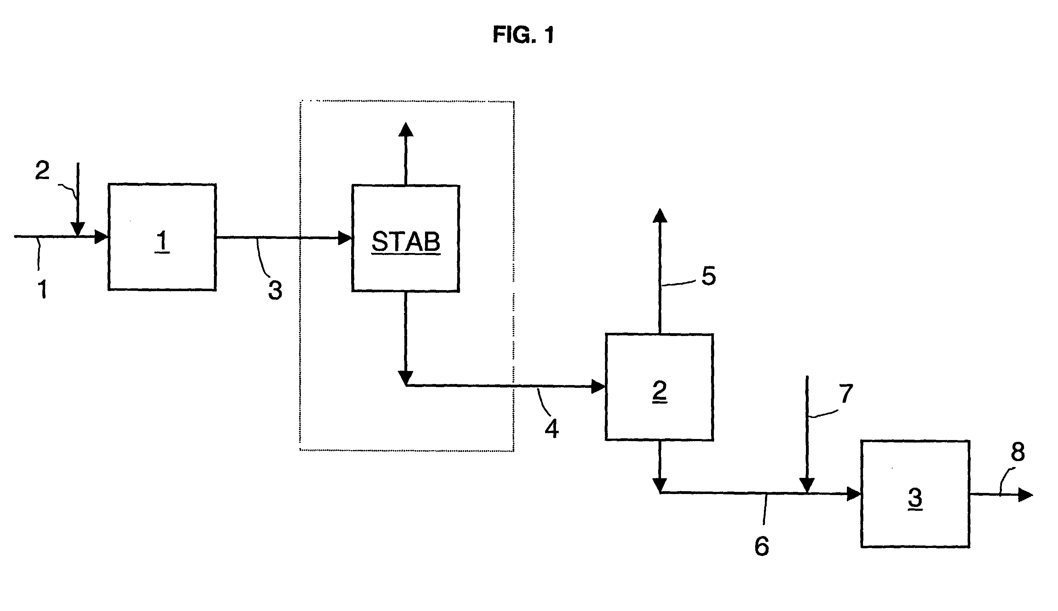

L'invention concerne également une installation (illustrée figure 1) utilisée pour mettre en oeuvre le procédé décrit ci-dessus.

Elle comporte successivement :
1) une zone 1 d'hydrogénation sélective avec isomérisation du butène-1 en butènes-2, ladite zone comportant au moins un moyen 1 pour l'introduction de la coupe à convertir, au moins un moyen 3 pour la sortie de l'effluent et au moins un moyen 2 pour l'introduction de l'hydrogène, ladite zone comportant également au moins un lit de catalyseur qui comprend de préférence au moins un métal choisi dans le groupe formé par le nickel, le palladium et le platine, déposé sur un support ;
2) une zone 2 de séparation, dans une colonne intégrant l'hydro-isomérisation des n-butènes (le butène-1 restant après l'étape 1 en butènes-2), comportant au moins un moyen 3 pour l'introduction de l'effluent issu de la zone 1, au moins un moyen 5 pour la sortie de l'isobutène, au moins un moyen 4 pour la sortie des butènes-2 et du n-butane ; et
3) une zone 3 de métathèse contenant au moins un catalyseur de préférence à base d'oxyde de rhénium déposé sur un support, et comprenant au moins un moyen 4 d'introduction de l'effluent issu de la zone 2, au moins un moyen 6 d'introduction de l'éthylène et au moins un moyen 7 pour la sortie du propylène.

De façon particulièrement intéressante, la coupe C₄ provient d'une zone de vapocraquage en amont, le moyen d'introduction de la coupe à convertir dans la zone 1 étant relié à ladite zone de vapocraquage, et le moyen d'introduction de l'éthylène dans la zone 4 étant relié à ladite zone de vapocraquage.

L'exemple suivant illustre l'invention sans en limiter la portée.

### EXEMPLE 1

Une coupe C₄ en sortie de vapocraqueur présente la composition indiquée dans le tableau 1 (flux 1). Dans ce tableau, les abréviations ont la signification suivante : MAPD = méthyl-acétylène + propadiène,
BBV = butadiène-1,2 + butyne-1 + vinyl-acétylène.

La coupe C₄ à traiter est d'abord soumise à un traitement d'hydrogénation et d'hydroisomérisation. Elle est introduite en continu, avec le débit massique indiqué dans le tableau 1, et sous une pression de 2 MPa, dans un premier réacteur comprenant un lit fixe de 2,6 T d'un catalyseur constitué par du palladium sur alumine, sulfuré au préalable. De l'hydrogène (mélangé à du méthane) est également injecté dans ce réacteur, comme indiqué dans le tableau 1 (flux 2). Afin de limiter l'augmentation de température dans le lit catalytique, la charge est mélangée avec l'effluent du réacteur dans un ratio 1 pour 20 avant traitement. L'effluent de ce premier réacteur est ensuite traité dans un réacteur de finition chargé avec 2,5 T du même catalyseur. A la sortie (tableau 1, flux 3), la coupe est débarrassée des composés acétyléniques, et le butadiène a été transformé essentiellement en butènes, qui sont en majorité des butènes-2, le butène-1 ayant été isomérisé. La coupe est alors traitée dans une colonne de stabilisation, où l'hydrogène résiduel et le méthane sont séparés. Après ce traitement, la coupe a la composition du flux 4 (tableau 1).

Dans la deuxième étape, la coupe C₄ hydro-isomérisée est soumise à un fractionnement dans une colonne intégrant l'hydro-isomérisation des n-butènes (du butène-1 restant après l'étape 1 en butènes-2). Cette colonne comporte 130 plateaux, est alimentée au plateau 90, et est équipée de trois réacteurs accolés chargés avec le même catalyseur que celui utilisé dans la première étape, et dont l'entrée et la sortie sont reliées directement à la colonne, respectivement au niveau des plateaux 10-11, 25-26 et 39-40. Le taux de reflux et les températures sont ajustés pour obtenir en tête un flux d'isobutène quasiment pur.

Dans la troisième étape, la fraction de pied de distillation qui contient principalement du butène-2 (composition: flux 6 du tableau 1) est mise en réaction avec de l'éthylène (composition globale : flux 6 + 7 du tableau 1) sur un catalyseur de métathèse, constitué par de l'oxyde de rhénium sur alumine gamma (8 % en poids en rhénium métal), préparé selon les enseignements du brevet US-A-4 795 734. La coupe C₄ est mélangée à l'entrée du réacteur de métathèse avec de l'éthylène d'appoint, ainsi qu'avec des flux de recyclage d'éthylène et de butènes. Ce réacteur fonctionne en lit mobile, comme décrit dans le brevet FR-B-2 608 595, à une température de 35 °C et sous une pression de 3,5 MPa, et il est couplé avec un régénérateur fonctionnant à 550 °C sous pression atmosphérique. Le catalyseur est soutiré à intervalles de temps réguliers en bas du réacteur et transféré vers le régénérateur, d'où il est renvoyé en haut du réacteur, les transferts se faisant à travers des sas tampons. En sortie de réacteur, l'éthylène non converti est fractionné dans une première colonne de distillation et recyclé. Une deuxième colonne de distillation sépare le propylène et les hydrocarbures en C₄ non convertis qui sont également recyclés. La composition de l'effluent de métathèse est indiquée dans le tableau 1, flux 8.

Le bilan global de la transformation s'établit comme suit. Pour 100 parties en poids (pp) de coupe C₄ sortie du vapocraqueur, on consomme 1,6 pp d'hydrogène et 29,5 pp d'éthylène, et on produit 27 pp d'isobutène haute pureté et 88,5 pp de propylène qualité « polymérisation ».

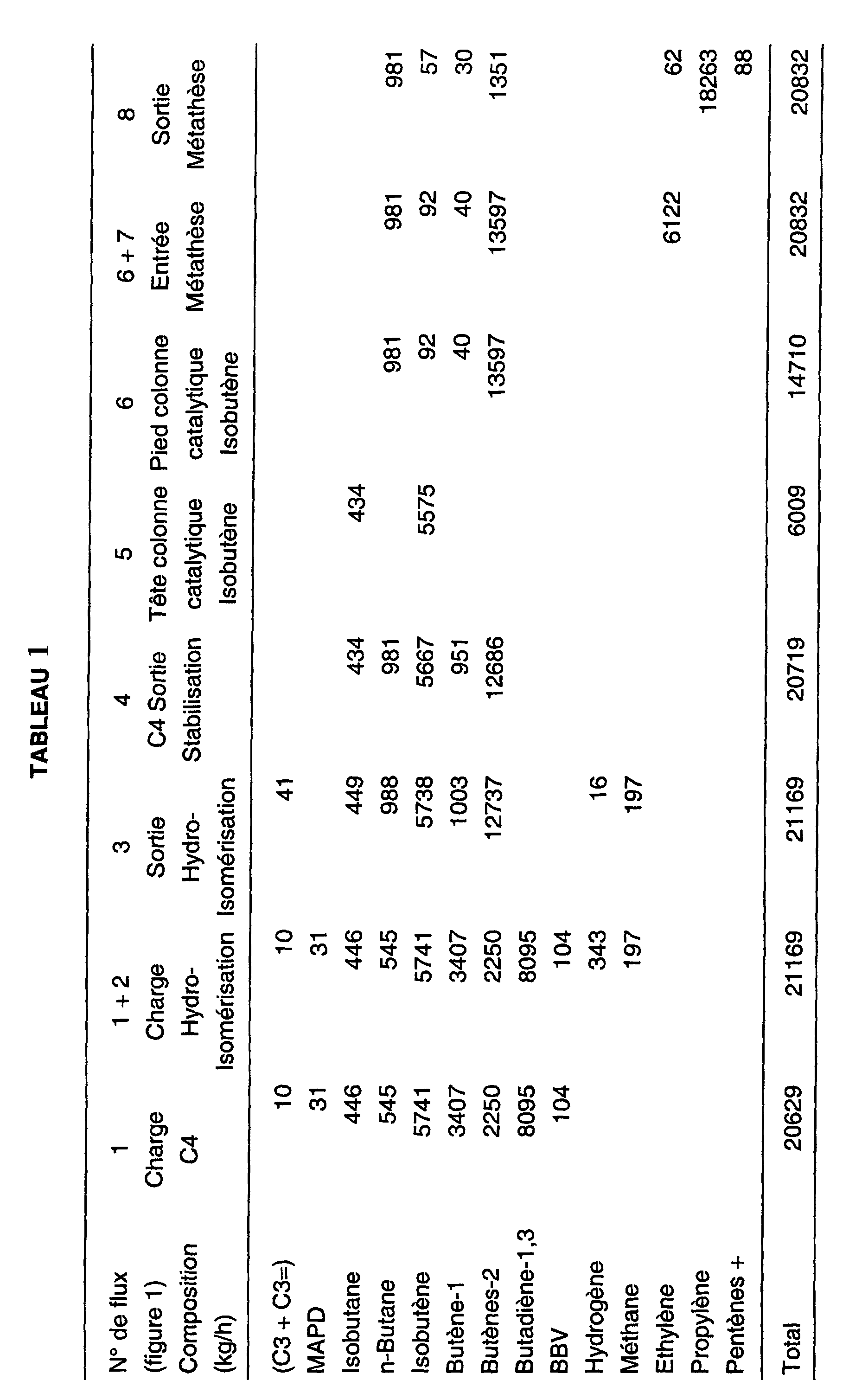

## Revendications

1. Procédé de conversion d'une coupe C₄ oléfinique en isobutène de haute pureté et en propylène, ladite coupe contenant notamment des dioléfines, du butène-1, des butènes-2, de l'isobutène et des impuretés acétyléniques, ledit procédé comportant les étapes suivantes, se déroulant successivement :
1) l'hydrogénation sélective des dioléfines et des impuretés acétyléniques avec isomérisation du butène-1 en butènes-2 en présence d'un catalyseur, de façon à obtenir un effluent contenant des n-butènes dont le ratio correspond à l'équilibre thermodynamique et de l'isobutène, et ne contenant pratiquement pas de dioléfines ni de composés acétyléniques ;
2) la séparation, par distillation, d'une coupe de tête contenant l'isobutène et d'une coupe de pied contenant essentiellement les butènes-2 et le butane ; et
3) la métathèse de la coupe butènes-2 issue de l'étape précédente avec de l'éthylène, en présence d'un catalyseur, de façon à obtenir un effluent contenant du propylène, la métathèse étant suivie d'une séparation du propylène,
ledit procédé étant **caractérisé en ce que** l'étape 2 est mise en oeuvre dans une colonne intégrant l'hydro-isomérisation du butène-1 restant en butènes-2.

2. Procédé selon la revendication 1, **caractérisé en ce que** l'étape 1 est effectuée par passage de ladite coupe en phase liquide sur un catalyseur comprenant au moins un métal choisi dans le groupe formé par le nickel, le palladium et le platine, déposé sur un support, à une température de 20 à 200 °C, une pression de 0,1 à 5 MPa, une vitesse spatiale de 0,5 à 10 h⁻¹, avec un rapport molaire H₂ / dioléfines de 0,5 à 5.

3. Procédé selon la revendication 1 ou 2, **caractérisé en ce que** le catalyseur de l'étape 1 contient de 0,05 à 10 % en poids de soufre.

4. Procédé selon l'une des revendications 1 à 3, **caractérisé en ce que** le catalyseur de l'étape 1 a été traité, avant son chargement dans le réacteur d'hydrogénation, par au moins un composé soufré dilué dans un solvant, et que le catalyseur obtenu, contenant de 0,05 à 10 % en poids de soufre est chargé dans le réacteur et activé sous atmosphère neutre ou réductrice à une température de 20 à 300 °C, une pression de 0,1 à 5 MPa et une VVH de 50 à 600 h⁻¹, et que la charge est mise au contact dudit catalyseur activé.

5. Procédé selon l'une des revendications 1 à 4, **caractérisé en ce que** le catalyseur de l'étape 1 est constitué par du palladium déposé sur de l'alumine, et du soufre.

6. Procédé selon l'une des revendications 1 à 5, **caractérisé en ce que** la colonne intégrant l'hydro-isomérisation du butène-1 restant en butènes-2 comporte, intérieurement ou extérieurement, une ou plusieurs charges d'un catalyseur tel que celui mis en oeuvre dans l'étape 1.

7. Procédé selon l'une des revendications 1 à 6, **caractérisé en ce que** la métathèse est réalisée dans l'étape 3 en présence d'un catalyseur comportant au moins un oxyde de rhénium déposé sur un support, à une température de 0 à 200 °C et à une pression au moins égale à la tension de vapeur du mélange réactionnel à la température de réaction,

8. Procédé selon la revendication 7, **caractérisé en ce que** ledit catalyseur contient de l'oxyde de rhénium à raison de 0,01 à 20 % en poids exprimé en rhénium métallique, déposé sur un support contenant au moins 75 % en poids d'alumine et de 0,01 à 30 % en poids d'au moins un oxyde d'un métal choisi dans le groupe formé par le niobium et le tantale.

9. Procédé selon l'une des revendications 1 à 8, **caractérisé en ce que** la métathèse est effectuée avec un catalyseur en lit mobile.

10. Procédé selon l'une des revendications 1 à 9, **caractérisé en ce que** la coupe C4 à traiter est une coupe de vapocraquage, et l'éthylène utilisé dans l'étape de métathèse provient de l'opération de vapocraquage.

11. Procédé selon l'une des revendications 2 à 10, **caractérisé en ce que** la colonne dans laquelle est effectuée l'étape 2 comporte, intérieurement ou extérieurement, au moins une charge d'un catalyseur défini de la même manière que celui mis en oeuvre dans l'étape 1.

12. Installation pour la conversion d'une coupe C₄ oléfinique en isobutène et en propylène, **caractérisée en ce qu'**elle comporte successivement :
1) une zone 1 d'hydrogénation sélective avec isomérisation du butène-1 en butène-2, ladite zone comportant au moins un moyen 1 pour l'introduction de la coupe à convertir, au moins un moyen 3 pour la sortie de l'effluent et au moins un moyen 2 pour l'introduction de l'hydrogène, ladite zone comportant également au moins un lit de catalyseur ;
2) une zone 2 de séparation, comportant au moins un moyen 3 pour l'introduction de l'effluent issu de la zone 1, au moins un moyen 5 pour la sortie de l'isobutène et du butène-1, au moins un moyen 4 pour la sortie du butène-2 et du n-butane ; et
3) une zone 3 de métathèse contenant au moins un lit de catalyseur et comprenant au moins un moyen 4 d'introduction de l'effluent issu de la zone 2, au moins un moyen 7 d'introduction de l'éthylène et au moins un moyen 8 pour la sortie du propylène ;
ladite zone de séparation 2 consistant en une colonne intégrant une zone d'hydro-isomérisation du butène-1 en butène-2.

13. Installation selon la revendication 12, **caractérisée en ce que** la zone de métathèse contient un lit mobile de catalyseur.

14. Installation selon l'une des revendications 12 et 13, **caractérisée en ce que** le moyen d'introduction de la coupe C₄ à convertir est relié à une zone de vapocraquage, et **en ce que** le moyen d'introduction de l'éthylène dans la zone de métathèse est relié à la zone de vapocraquage.

## Patentansprüche

1. Verfahren zur Umwandlung einer olefinischen C₄-Fraktion zu Isobuten hoher Reinheit und zu Propylen, wobei die Fraktion vor allem Olefine, 1-Buten, 2-Butene, Isobuten und acetylenische Verunreinigungen enthält, welches Verfahren die folgenden Stufen umfasst, die aufeinanderfolgend ablaufen:
1) die selektive Hydrierung der Diolefine und der acetylenischen Verunreinigungen mit isomerisierung des 1-Butens zu 2-Butenen in Gegenwart eines Katalysators, derart, dass ein Abstrom erhalten wird, der n-Butene, deren Verhältnis dem thermodynamischen Gleichgewicht entspricht und Isobuten enthält und praktisch weder Diolefine noch acetylenische Verbindungen enthält;
2) die Trennung durch Destillation, von einer Kopffraktion, die Isobuten enthält, und einer Bodenfraktion, die im wesentlichen 2-Butene und Butan enthält; und
3) die Metathesis der 2-Butenefraktion aus der vorhergehenden Stufe mit Ethylen in Gegenwart eines Katalysators, derart, dass ein Abstrom erhalten wird, der Propylen enthält, wobei die Metathesis von einer Trennung des Propylens gefolgt ist,
wobei das Verfahren **dadurch gekennzeichnet ist, dass** die Stufe 2 in einer Kolonne durchgeführt wird, die die Hydrierisomerisierung des verbleibenden 1-Butens zu 2-Butenen integriert.

2. Verfahren nach Anspruch 1, **dadurch gekennzeichnet, dass** die Stufe 1 durch Durchgang der Fraktion in Flüssigphase über einen Katalysator durchgeführt wird, der wenigstens ein Metall, gewählt aus der Gruppe, die gebildet wird durch Nickel, Palladium und Platin, abgeschieden auf einen Träger umfasst, bei einer Temperatur von 20 bis 200°C, einem Druck von 0,1 bis 5 MPa, einer Raumgeschwindigkeit von 0,5 bis 10 h⁻¹ mit einem molaren Verhältnis H₂/Diolefine von 0,5 bis 5.

3. Verfahren nach Anspruch 1 oder 2, **dadurch gekennzeichnet, dass** der Katalysator der Stufe 1 0,05 bis 10 Gew.-% Schwefel enthält.

4. Verfahren nach einem der Ansprüche 1 bis 3, **dadurch gekennzeichnet, dass** der Katalysator der Stufe 1 vor seiner Beschickung in den Hydrierungsreaktor durch wenigstens eine in einem Lösungsmittel verdünnte schwefelhaltige Verbindung behandelt worden ist, und dass der erhaltene Katalysator, der 0,05 bis 5 Gew.-% Schwefel enthält, in den Reaktor geladen und unter neutraler oder reduzierender Atmosphäre bei einer Temperatur von 20 bis 300°C, einem Druck von 0,1 bis 5 MPa und einer VVH von 50 bis 600 h⁻¹ aktiviert wird und dass die Charge mit dem aktivierten Katalysator kontaktiert wird.

5. Verfahren nach einem der Ansprüche 1 bis 4, **dadurch gekennzeichnet, dass** der Katalysator der Stufe 1 aus Palladium, abgeschieden auf Aluminiumoxid und aus Schwefel besteht.

6. Verfahren nach einem der Ansprüche 1 bis 5, **dadurch gekennzeichnet, dass** die Kolonne, die die Hydrierisomerisierung des verbleibenden 1-Butens zu 2-Butenen integriert, innen oder außen eine oder mehrere Beschickungen eines Katalysators wie jenem in der Stufe 1 eingesetzten umfasst.

7. Verfahren nach einem der Ansprüche 1 bis 6, **dadurch gekennzeichnet, dass** die Metathesis in der Stufe 3 in Gegenwart eines Katalysators durchgeführt wird, der wenigstens ein Rheniumoxid, abgeschieden auf einem Träger umfasst, bei einer Temperatur von 0 bis 200°C und einem Druck wenigstens gleich der Dampfspannung des Reaktionsgemischs bei Reaktionstemperatur.

8. Verfahren nach Anspruch 7, **dadurch gekennzeichnet, dass** der Katalysator Rheniumoxid im Verhältnis von 0,01 bis 20 Gew.-% ausgedrückt in metallischem Rhenium, abgeschieden auf einem Träger enthält, der wenigstens 75 Gew.-% Aluminiumoxid und 0,01 bis 30 Gew.-% wenigstens eines Oxids eines Metalls enthält, das gewählt ist aus der Gruppe, die gebildet wird durch Niob und Tantal.

9. Verfahren nach einem der Ansprüche 1 bis 8, **dadurch gekennzeichnet, dass** die Metathesis mit einem Katalysator im beweglichen Bett durchgeführt wird.

10. Verfahren nach einem der Ansprüche 1 bis 9, **dadurch gekennzeichnet, dass** die zu behandelnde olefinische C₄-Fraktion eine Dampfcrackfraktion ist und das in der Metathesisstufe verwendete Ethylen von dem Dampfcrackvorgang kommt.

11. Verfahren nach einem der Ansprüche 2 bis 10, **dadurch gekennzeichnet, dass** die Kolonne, in der die Stufe 2 durchgeführt wird innen oder außen wenigstens eine Beschickung eines Katalysators umfasst, der wie jener in der Stufe 1 eingesetzte definiert ist.

12. Anlage zur Umwandlung einer olefinischen C₄-Fraktion zu Isobuten und zu Propylen, **dadurch gekennzeichnet, dass** sie aufeinanderfolgend umfasst:
1) eine selektive Hydrierungszone 1 mit Isomerisierung des 1-Butens zu 2-Buten, wobei die Zone umfasst wenigstens ein Mittel 1 zur Einführung der umzuwandelnden Fraktion, wenigstens ein Mittel 3 zum Austritt des Abstroms und wenigstens ein Mittel 2 zur Einführung des Wasserstoffs, wobei diese Zone auch wenigstens ein Katalysatorbett umfasst;
2) eine Trennzone 2, die wenigstens ein Mittel 3 zur Einführung des Abstroms aus der Zone 1, wenigstens ein Mittel 5 zum Austritt des Isobutens und des 1-Butens, wenigstens ein Mittel 4 zum Austritt des 2-Butens und n-Butans umfasst; und
3) eine Metathesiszone 3, die wenigstens ein Katalysatorbett enthält und wenigstens ein Mittel 4 zur Einführung des Abstroms aus der Zone 2, wenigstens ein Mittel 7 zur Einführung des Ethylens und wenigstens ein Mittel 8 zum austritt des Propylens umfasst;
wobei die Trennzone 2 aus einer Kolonne besteht, die die Hydrierisomerisierung des verbleibenden 1-Butens zu 2-Buten integriert.

13. Anlage nach Anspruch 12, **dadurch gekennzeichnet, dass** die Metathesiszone ein bewegliches Katalysatorbett enthält.

14. Anlage nach einem der Ansprüche 12 und 13, **dadurch gekennzeichnet, dass** das Mittel zur Einführung der umzuwandelnden C₄-Fraktion mit einer Dampfcrackzone verbunden ist und dadurch, dass das Mittel zur Einführung des Ethylens in die Metathesiszone mit der Dampfcrackzone verbunden ist.

## Claims

1. Process for converting an olefinic C₄ fraction into high-purity isobutene and into propylene, whereby said fraction contains in particular diolefins, butene-1, butenes-2, isobutene and acetylenic impurities, and whereby said process comprises the following stages that take place successively:
1) the selective hydrogenation of diolefins and acetylenic impurities with isomerization of butene-1 into butenes-2 in the presence of a catalyst, in order to obtain an effluent that contains n-butenes, whose ratio corresponds to the thermodynamic equilibrium and isobutene, and that contains virtually no diolefins or acetylenic compounds;
2) the separation, by distillation, of a top fraction that contains isobutene and a bottom fraction that contains essentially butenes-2 and butane; and
3) the metathesis of the butenes-2 fraction that is obtained from the preceding stage with the ethylene, in the presence of a catalyst, so as to obtain an effluent that contains propylene, whereby the metathesis is followed by a separation of the propylene;
whereby said process is **characterized in that** stage 2 is implemented in a column that integrates the hydroisomerization of butene-1 that remains to butenes-2.

2. Process according to claim 1, **characterized in that** stage 1 is carried out by running said fraction in the liquid phase over a catalyst that comprises at least one metal that is selected from the group that is formed by nickel, palladium and platinum, deposited on a substrate, at a temperature of 20 to 200°C, a pressure of 0.1 to 5 MPa, a volumetric flow rate of 0.5 to 10 h⁻¹, with an H₂/diolefin molar ratio of 0.5 to 5.

3. Process according to claim 1 or 2, **characterized in that** the catalyst of stage 1 contains 0.05 to 10% by weight of sulfur.

4. Process according to one of claims 1 to 3, **characterized in that** the catalyst of stage 1 was treated, before being loaded into the hydrogenation reactor, by at least one sulfur-containing compound that is diluted in a solvent, **in that** the catalyst that is obtained and that contains 0.05 to 10% by weight of sulfur is loaded into the reactor and activated under a neutral atmosphere or a reducing atmosphere at a temperature of 20 to 300°C, a pressure of 0.1 to 5 MPa and a VVH of 50 to 600 h⁻¹, and **in that** the feedstock is brought into contact with said activated catalyst.

5. Process according to one of claims 1 to 4, **characterized in that** the catalyst of stage 1 consists of palladium that is deposited on alumina, and sulfur.

6. Process according to one of claims 1 to 5, **characterized in that** the column that integrates the hydroisomerization of butene-1 that remains to butenes-2 comprises, on the inside or outside, one or more loads of a catalyst such as the one that is used in stage 1.

7. Process according to one of claims 1 to 6, **characterized in that** the metathesis is carried out in stage 3 in the presence of a catalyst that comprises at least one rhenium oxide that is deposited on a substrate, at a temperature of 0 to 200°C and at a pressure that is at least equal to the vapor tension of the reaction mixture at the reaction temperature.

8. Process according to claim 7, **characterized in that** said catalyst contains rhenium oxide at a rate of 0.01 to 20% by weight expressed in metallic rhenium, deposited on a substrate that contains at least 75% by weight of alumina and 0.01 to 30% by weight of at least one oxide of a metal that is selected from the group that is formed by niobium and tantalum.

9. Process according to one of claims 1 to 8, **characterized in that** the metathesis is carried out with a moving-bed catalyst.

10. Process according to one of claims 1 to 9, **characterized in that** the C₄ fraction that is to be treated is a steam-cracking fraction, and the ethylene that is used in the metathesis stage is obtained from the steam-cracking operation.

11. Process according to one of claims 2 to 10, **characterized in that** the column in which stage 2 is implemented comprises, on the inside or outside, at least one load of a catalyst defined in the same manner as the one that is used in stage 1.

12. Installation for the conversion of an olefinic C₄ fraction into isobutene and into propylene, **characterized in that** it successively comprises:
1) a selective hydrogenation zone 1 with isomerization of butene-1 into butene-2, whereby said zone comprises at least one means 1 for introducing the fraction that is to be converted, at least one means 3 for the output of the effluent and at least one means 2 for the introduction of hydrogen, whereby said zone also comprises at least one catalyst bed;
2) a zone 2 for separation that comprises at least one means 3 for introducing the effluent that is obtained from zone 1, at least one means 5 for the output of isobutene and butene-1, at least one means 4 for the output of butene-2 and n-butane; and
3) a metathesis zone 3 that contains at least one catalyst bed and that comprises at least one means 4 for introducing the effluent that is obtained from zone 2, at least one means 7 for introducing ethylene and at least one means 8 for the output of propylene,
whereby said separation zone 2 consists of a column that integrates a zone for hydroisomerization from butene-1 to butene-2.

13. Installation according to claim 12, **characterized in that** the metathesis zone contains a catalyst moving bed.

14. Installation according to one of claims 12 and 13, **characterized in that** the means for introducing the C₄ fraction that is to be converted is connected to a steam-cracking zone and **in that** the means for introducing ethylene into the metathesis zone is connected to the steam-cracking zone.
